# EUROPÄISCHE PATENTSCHRIFT

(11) **EP 2 234 988 B1**
(45) Veröffentlichungstag und Bekanntmachung des Hinweises auf die Patenterteilung: **16.10.2013**
(21) Anmeldenummer: 08860917.7
(22) Anmeldetag: 04.12.2008
(51) Int. Cl.: C07D 251/34, C08G 18/00

(54) **VERFAHREN ZUR HERSTELLUNG MONOMERENARMER ORGANISCHER POLYISOCYANATE**
METHOD FOR PRODUCING LOW-MONOMER ORGANIC POLYISOCYANATE
PROCÉDÉ DE PRODUCTION DE POLYISOCYANATES ORGANIQUES PAUVRES EN MONOMÈRES

(30) Priorität: 18.12.2007 DE 102007060791
(43) Veröffentlichungstag der Anmeldung: 06.10.2010
(73) Patentinhaber: Bayer Intellectual Property GmbH, 40789 Monheim (DE)
(72) Erfinder: BRAHM, Martin, 51519 Odenthal (DE); RICHTER, Frank, 51373 Leverkusen (DE); VOIGT, Thomas, 40764 Langenfeld (DE); HALPAAP, Reinhard, 51519 Odenthal (DE)
(74) Vertreter: BIP Patents
(86) Internationale Anmeldenummer: PCT/EP2008/010281
(87) Internationale Veröffentlichungsnummer: WO 2009/077085

(56) Entgegenhaltungen:
- EP-A- 0 727 453
- EP-A- 0 798 299
- EP-A- 0 818 485
- EP-A- 1 378 529
- LAAS H J ET AL: "ZUR SYNTHESE ALIPHATISCHER POLYISOCYANATE-LACKPOLYISOCYANATE MIT BIURET-, ISOCYANURAT- ODER URETDIONSTRUKTUR. ÖTHE SYNTHESIS OF ALIPHATIC POLYISOCYANATES CONTAINING BIURET, ISOCYANURATE OR URETDIONE BACKBONES FOR USE IN COATINGS" JOURNAL FUER PRAKTISCHE CHEMIE, WILEY VCH, WEINHEIM, Bd. 336, Nr. 3, 1. Januar 1994 (1994-01-01), Seiten 185-200, XP000441642 ISSN: 1436-9966 in der Anmeldung erwähnt

## Beschreibung

Die Erfindung betrifft ein neues technisches Verfahren zur Herstellung monomerenarmer organischer Polyisocyanate durch Oligomerisierung organischer Diisocyanate in einem Zweiphasensystem.

Die Herstellung oligomerer Polyisocyanate ist seit langem bekannt (H. J. Laas et al, J. prakt. Chem. 336, 185-200 (1994); EP 755 954, EP 798 299, EP 508 313). So sind verschiedenste Oligomerisierungsreaktionen beschrieben bei denen ein Teil der vorhandenen Isocyanatgruppen bis zu einem vorgegebenen Umsatz abreagiert und im Anschluss restlich vorhandene Monomere z. B. durch Destillation entfernt werden. Als Oligomerisierungsreaktionen haben sich mit der katalytischen Trimerisierung, Dimerisierung, Carbodiimidisierung, Allophanatisieung, mit der Biuretisierung, Harnstoffbildung und Urethanisierung organischer Diisocyanate unterschiedlichste Bildungsmechanismen etabliert. Üblicherweise werden in Substanz ohne Löse- oder Verdünnungs-mittel 10 bis 40 % der Isocyanatgruppen der Diisocyanatkomponente der Oligomerisierungsreaktion unterzogen. Nach Abstoppen der Reaktion durch Desaktivierung des Katalysators verbleibt hierbei zumeist ein größerer Teil des Ausgangsisocyanats in der Reaktionsmischung zurück. Eine nachfolgende destillative Abtrennung beispielsweise in Dünnschichtverdampfern führt letztlich zu Produkten mit einem Restmonomergehalt von unter 0,5 bzw. zum Teil unter 0,1 %.

Nachteilig bei Reaktionen ohne Lösemittel ist das hohe Reaktionspotential, das bei den zumeist stark exothermen Reaktionen (damit starke Temperaturerhöhung der Reaktionsmischung) zu einem unkontrollierten Reaktionsverlauf führen kann und somit verfahrenstechnisch durch komplizierte Prozessabschaltungen abgesichert werden muss. Des weiteren steigt die Viskosität im Verlaufe der Reaktion deutlich an, wodurch sich erhebliche verfahrenstechnische Probleme ergeben. Alle Apparate müssen für einen weiten Temperatur- und Viskositätsbereich ausgelegt sein und arbeiten somit nicht im Optimalbereich.

Wird das Reaktionspotential durch Zusatz größerer Mengen an Lösemittel herabgesetzt, verbleibt dieses in der Reaktionsmischung und muss, wenn überhaupt möglich, aufwändig destillativ entfernt werden.

Werden entsprechende Reaktionen kontinuierlich z. B. in einem oder mehreren Rührkesseln durchgeführt, muss die Reaktion - aufgrund von Rückvermischungen in den Kaskadenreaktoren gegenüber einer diskontinuierlichen Reaktion frühzeitiger abgebrochen werden, um die gleichen Kenndaten der resultierenden Polyisocyanate zu erzielen. Dieser Umsatzverlust (erhöhte Menge an nicht umgesetztem Ausgangsdiisocyanat) muss durch eine höhere Destillationsleistung und einen erheblichen Mehraufwand kompensiert werden. Günstiger wäre hier die Batch-Reaktion ohne Zufuhrung von frischem Diisocyanat während der Oligomerisierung. Limitiert wird diese Art der Reaktionsführung und damit die Batch-Größe jedoch durch die notwendige Wärmeabfuhr und die technisch realisierbaren Kühlsysteme.

Bei der ebenfalls möglichen Durchführung von Isocyanatreaktionen in einem Rohrreaktor kommt es aufgrund der großen Oberfläche der Rohre zu starken Ablagerungs- und Verschmutzungserscheinungen, die zur Notwendigkeit einer vorzeitigen Reinigung der Anlage führen kann. Die unvermeidliche Belagbildung führt zu schlechten Temperaturübergängen an den Rohrwandungen und somit zu stärkeren Temperaturinhomogenitäten. Dies beeinflußt sowohl die Qualität (z. B. Farbe) als auch die Ausbeute des herzustellenden Produkts.

Es war daher Aufgabe der vorliegenden Erfindung, ein universelles Verfahren zur Herstellung monomerenarmer Polyisocyanate bereitzustellen, das diese Nachteile beseitigt.

Diese Aufgabe konnte mit dem nachstehend näher beschriebenen erfindungsgemäßen Verfahren gelöst werden.

Gegenstand der Erfindung ist ein Verfahren zur Herstellung von Polyisocyanaten, dadurch gekennzeichnet, dass
A) eine Isocyanatkomponente, welche zu mindestens 60 Gew.-% aus einem oder mehreren Diisocyanaten und zu maximal 40 Gew.-% aus einem oder mehreren Monoisocyanaten und/oder einem oder mehreren Isocyanaten mit einer Funktionalität ≥ 3 besteht
B) ggf. mit einem Katalysator B1) und/oder ggf. einer Reaktivkomponente B2)
in Form von Tropfen in einem Verdünnungsmittel C) bei einer Temperatur von 0 bis 200°C unter Oligomerisierung zur Reaktion gebracht wird, dann eine Phasentrennung erfolgt, vor, während oder nach der Phasentrennung die Reaktion ggf. durch Zusatz eines Katalysatorgifts D) unterbrochen wird, und anschließend das in der Isocyanatphase enthaltene Polyisocyanat vom überschüssi-gen monomeren Isocyanat bis auf einen Restgehalt von weniger als 1,0 Gewichts-% befreit wird, wobei die Restlöslichkeit der Isocyanatkomponente im Verdünnungsmittel unter 10 Gewichts-% liegt und als Verdunnungsmittel C) Alkane eingesetzt werden, die fluoriert oder perfluoriert sind.

Als Isocyanatkomponente A) sind alle Isocyanatgruppen aufweisenden organischen Verbindungen und Gemische zu verstehen, die mindestens einen Gehalt an Diisocyanaten von 60 Gewichts-%, vorzugsweise 80 Gewichts-% und besonders bevorzugt von 95 Gewichts-% enthalten. Ganz besonders bevorzugt enthält die Isocyanatkomponente ausschließlich Diisocyanate, wobei insbesondere nur ein Diisocyanat eingesetzt wird.

Als einsetzbare Isocyanate in der Isocyanatkomponente A) beispielhaft genannt seien an sich bekannte Monoisocyanate mit aliphatisch, cycloaliphatisch, araliphatisch oder aromatisch gebundenen Isocyanatgruppen wie z. B. Stearylisocyanat, Naphthylisocyanat, Diisocyanate mit aliphatisch, cycloaliphatisch, araliphatisch und/oder aromatisch gebundenen Isocyanatgruppen, wie z. B. 1,4-Diisocyanatobutan, 1,6-Diisocyanatohexan (HDI), 2-Methyl-1,5- diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 2,4'- und 4,4'-Diisocyanatodicyclohexylmethan, 1-Isocyanato-1-methyl-4(3)-isocyanatomethylcyclohexan (IMCI), Bis-(isocyanatomethyl)-norbornan, 2,4- und 2,6-Diisocyanatotoluol (TDI), 2,4'- und 4,4'-Diisocyanatodiphenylmethan und höhere Homologe, 1,5-Diisocyanatonaphthalin, "Dipropylenglycoldiisocyanat" (Isomere aus 2-(2-Isocyanatopropoxy)-1-propylisocyanat, 1,1'-Oxy-di-2-propylisocyanat und 2,2'-Oxydi-1-propylisocyanat), Triisocyanate und/oder höherfunktionelle Isocyanate wie z. B. 4-Isocyanatomethyl-1,8-octandiisocyanat (Nonantriisocyanat), 1,6,11-Undecantriisocyanat oder beliebige Gemische solcher Isocyanatverbindungen.

Es können beliebige Mischungen der genannten Isocyanate innerhalb der oben angegebenen Gewichtsgrenzen eingesetzt werden.

Vorzugsweise werden Isocyanatverbindungen eingesetzt, die aliphatisch gebundene Isocyanatgruppen enthalten. Besonders bevorzugt werden als Isocyanatkomponente A) HDI, IPDI 2,4'-und 4,4'-Diisocyanatodicyclohexylmethan eingesetzt.

Als Katalysator B1) können beliebige Trimerisierungs-, Dimerisierungs-, Carbodiimidisierungs-, Allophanatisierungs-katalysatoren eingesetzt werden, wie sie beispielhaft in H. J. Laas et al, J. prakt. Chem. 336, 185-200 (1994) und dort zitierter Literatur; EP 755 954, EP 798 299 genannt sind. Beispielhaft seien als Katalysatoren B1) genannt: Blei-II-acetat, Blei-II-2-ethylhexanoat, Mannichbasen wie beispielsweise ein Umsetzungsprodukt aus Phenol mit Dimethylamin, tertiäre Amine wie Diazabicyclo[2.2.2]octan (DABCO), N,N,N',N'-Tetramethylethylendiamin, gegebenenfalls in Mischung mit Epoxiden, Alkali- und Erdalkaliverbindungen wie Oxide, Hydroxide, Carbonate, Alkoholate oder Phenolate, aber auch Metallsalze schwacher aliphatischer oder cyclo-aliphatischer Carbonsäuren, gegebenenfalls in Gegenwart komplexierend wirkender Kronenether oder linearer Polyether. Geeignete Katalysatoren B1) sind außerdem z. Bsp., Tris-(dimethylaminopropyl)-hexahydrotriazin (^{®}Polycat 41, Hersteller: Air Products), gegebenenfalls als Anlagerungsverbindung mit Phenolen oder Carbonsäuren. Gut geeignet als Katalysatoren B1) sind quarternäre Ammoniumbasen wie Trimethylbenzylammoniumhydroxid (^{®}Triton B, Hersteller: Merck KGaA, Germany), Methylcholin oder Hydroxyalkylammoniumcarboxylate oder quartäre Ammoniumfluoride. Neben den beispielhaft genannten Ammoniumsalzen sind auch entsprechende Phosphoniumsalze oder Phosphine als Katalysatoren B1) verwenddbar. Aminosilane sind eine weitere Verbindungsklasse als Katalysatoren B), z. Bsp. Hexamethyldisilazan (HMDS).

Die Katalysatoren werden ggf. gelöst in Lösemitteln oder Reaktivverbindungen wie z. B. Alkoho-len vor oder nach der Tropfenbildung zudosiert. Hierbei reicht der Gehalt bezogen auf die Isocyanatkomponente von 1 ppm bis 5 % bevorzugt 5 ppm bis 0,5 % und besonders bevorzugt 10 ppm bis 0,1 %. Üblicherweise wird der Katalysator kurz vor der Tropfenbildung in die Isocyanatkomponente eingebracht. Es ist jedoch auch möglich und vorteilhaft den Katalysator zunächst in dem Verdünnungsmittel zu lösen oder zu dispergieren (beispielsweise mittels Düsenstrahldispergator). Der Katalysator wandert dann fein verteilt aus dieser Phase in die Isocyanattropfen. Man kann jedoch auch so verfahren, dass zunächst eine heterogene Mischung aus Isocyanat und nichtlösendem Verdünnungsmittel C) hergestellt wird und zu der gerührten Isocyanat-Tröpfchen enthaltenden Mischung der Katalysator B1) zudosiert wird.

Anstelle von Katalysatoren B1) kann eine Oligomerisierung der Isocyanatkomponente A) auch durch Zusatz einer Reaktivkomponente B2), d.h. durch Zusatz von Wasser, niedermolekularen Alkoholen oder Aminen bzw. entsprechenden Mischungen mit 1 bis 6 funktionellen Einheiten erfolgen. Vorzugsweise werden Alkohole mit 1 bis 3 OH-Gruppen und einem Molekulargewicht zwischen 32 und 300 g/Mol verwendet. Als Amine kommen vorzugsweise Mono- und Diamine bis zu einem Molekulargewicht von 350 g/Mol zum Einsatz. Die Menge liegt hierbei bei bis zu 30 Gew.-%, vorzugsweise zwischen 1 Gewichts-% und 20 Gewichts-%, ganz besonders bevorzugt zwischen 2 Gew.-% und 10 Gew.-% bez. auf die Menge an Diisocyanatkomponente A).

Werden z. Bsp. durch Umsetzung mit Aminen als Reaktivkomponente B2) Biuret-Strukturen aufweisende Polyisocyanate hergestellt, so wird vorzugsweise das Amin in dem Verdünnungsmittel C) vordispergiert und dann mit dispergierten Isocyanttropfen zur Reaktion gebracht. Es ist jedoch ebenfalls möglich das Isocyanat im Verdünnungsmittel C) als Tröpfchen zu emulgieren und das Amin bei erhöhter Temperatur zu dieser heterogenen Mischung zuzugeben.

Aus Alkoholen entstehen während der Zweiphasenreaktion Urethane bzw. Allophanate; Amine führen zu Biuretstrukturen.

Bei dieser Vorgehensweise können neben den Reaktivkomponenten B2) natürlich auch Katalysatoren B1), die die Umsetzung von Isocyanaten mit Alkoholen zu Urethanen und/oder Allophanaten beschleunigen oder die die Umsetzung von Isocyanaten mit Aminen zu Biureten katalysieren mitverwendet werden. Hierbei finden als Allophanatisierungkatalysatoren die oben bereits beschriebenen Verbindungen Anwendung. Als Urethanisierungskatalysatoren können bekannte Verbindungen wie Amine, Metallsalze, wie z. Bsp. Sn-, Zn- oder Bi-Verbindungen eingesetzt werden, z. B. Dibutylzinndilaurat (DBTL) oder Bismuttrisneodecanoat (Coscat 83).

Die Isocyanattropfen können durch intesives Rühren oder aber auch wie z. B. bei Extraktionsverfahren üblich durch Verdüsung vor oder in das Verdünnungsmittel C) erzeugt werden. Zur Erzeugung feiner Tröpfchen eignet sich beispielweise eine Düsenstrahldispergierung. Die Tropfengröße reicht hierbei bis zu 5 mm Durchmesser. Vorzugsweise beträgt die Tropfengröße 0,1 µm - 1 mm, besonders bevorzugt 1 µm - 1 mm und ganz besonders bevorzugt 10 µm - 1 mm. In der Regel wird während der Reaktion intensiv gerührt um die Emulsion einige Zeit stabil zu halten, es ist aber ebenfalls möglich in einem Umpumpkreislauf die zweiphasige flüssige Mischung erneut zu verdüsen.

Verdünnungsmittel C) sind bei Reaktionsbedingungen flüssigen Verbindungen, die weder die Isocynanatkomponente A) noch das entstehende Polyisocyanat lösen. Die Restlöslichkeit der Isocyanatkomponente im Verdünnungsmittel liegt unter 10 Gewichts-% vorzugsweise unter 1 Gewichts-% und besonders bevorzugt unter 0,1 Gewichts-%. Umgekehrt liegt die Löslichkeit des Verdünnungsmittels in der Isocyanatkomponente A) und im Polyisocyanat unter 10 Gewichts-% vorzugsweise unter 1 Gewichts-% und besonders bevorzugt unter 0,1 Gewichts-%.

Es werden als hydrophobe Verbindungen , fluorhaltige Alkane und/oder perfluorierte Alkane als Verdünnungsmittel C eingesetzt. Ganz besonders bevorzugt ist Perfluoroktan.

Vorzugsweise sind die Verdünnungsmittel unreaktiv gegenüber der Isocyanatkomponente A).

Während der Reaktion wird ein Gewichts-Verhältnis von Isocyanatkomponente A) zu Verdünnungsmittel C) von 1:20 bis 5:1, vorzugsweise 1:10 bis 1:1 und besonders bevorzugt 1:5 bis 1:2 eingestellt.

Bei Reaktionsbedingungen muss sich die Dichte des Verdünnungsmittels C) von der Dichte der Isocyanatkomponente A) bzw. der entstandenen Reaktionsmischung unterscheiden.

Die Oligomerisierungsreaktion wird im Zweiphasensystem bei einer Temperatur von 0 bis 200 °C, vorzugsweise 20 bis 100 °C und besonders bevorzugt 40 bis 80 °C durchgeführt.

Gegebenenfalls wird die Reaktion durch Zusatz von Katalysatorgiften D) vor, während oder nach der Abtrennung des Verdünnungsmittels abgebrochen. Derartige Verbindungen sind beispielsweise beschrieben in H. J. Laas et al, J. prakt. Chem. 336, 185-200 (1994) und dort zitierter Literatur; EP 755 954, EP 798 299. Als Katalysatorgifte oder sogenannte Abstopper geeignet sind je nach Art der verwendeten Katalyse Säuren, Säurederivate, Alkylierungsmittel oder Alkohole, wie z. B. Phosphorsäure oder Phosphorigsäure oder deren Ester, Salzsäure, Carbonsäuren oder Carbonsäurechloride (z. B. Benzoylchlorid), Toluolsulfonsäuremethylester oder niedere Alkohole (z. B. Butanol). Bevorzugte Abstopper sind Phosphorsäure- oder Phosphorigsäureester. Neben der chemischen Abstoppung ist es jedoch ebenfalls möglich die Reaktion thermisch abzustoppen, indem man beispielsweise 30 min bis wenige Stunden auf Temperaturen oberhalb z. B. 60 oder 80 °C heizt und den Katalysator durch thermische Zersetzung desaktiviert. Vorzugsweise wird die Reaktion durch Zusatz von Katalysatorgiften gestoppt.

Nach der Reaktion und ggf. Stoppung durch Zusatz eines Katalysatorgifts erfolgt die Auftrennung der beiden Phasen in zwei separate Phasenbereiche durch Vereinigung der Tropfen. Dies kann z. B. durch eine Beruhigung und Auftrennung der Reaktionsmischung oder Separierung mittels halbdurchlässiger Trennschichten bewerkstelligt werden. Die so hergestellte und vom Verdünnungs-mittel separierte Rohware wird im Anschluss z. B. mittels Destillation, z. B. zweistufige Fallfilm- / Dünnschichtverdampfung, in Polyisocyanatkomponente und Ausgangsdiisocyanat getrennt. Hierbei liegt der verbleibende Anteil an monomerem Diisocyanat in der Polyisocyanatkomponente unter 1,0 Gewichts-% bevorzugt unter 0,5 Gewichts-% und besonders bevorzugt unter 0,15 Gewichts-%. Man kann aber ebenfalls ohne Phasenseparierung die vorliegende Rohmischung durch destillative Abtrennung des Verdünnungsmittels und nachfolgende destillative Abtrennung des monomeren Diisocyanats das Polyisocyanat isolieren.

Wird die Reaktion im Zweiphasensystem kontinuierlich betrieben, kann die Isocyanatkomponente A) beispielsweise durch Verdüsung von oben oder von unten in einer mit Verdünnungsmittel gefüllten Kolonne (Zylinder) in Tropfenform überführt werden. Die vorgegebene Reaktionstemperatur kann ggf. durch Umpumpung und Abscheidung des Verdünnungsmittels im Gegenstrom der sich bewegenden Tropfen mittels externer Kühlung bzw. Heizung einstellt werden. Der Katalysator bzw. die Reaktivkomponente kann zuvor durch Mischung in die Isocyanatkomponente eingebracht werden. Es ist jedoch auch möglich den Katalysator getrennt in dem Verdünnungsmittel zu lösen oder zu dispergieren.

Bei der kontinuierlichen Fahrweise erfolgt die Reaktion entlang der Kolonne, indem die Tropfen bedingt durch den Dichteunterschied gegenüber dem Verdünnungsmittel zu Boden fallen bzw. hochsteigen. An der oberen bzw. unteren Grenze fmdet dann eine Phasenseparation statt, von der aus die Polyisocyanat-Rohlösung zur weiteren Aufarbeitung und ggf. Stoppung entfernt werden kann. Die Phasentrennung kann auch in einem separaten Behälter kontinuierlich oder diskontinuierlich durchgeführt werden. Das ggf. verunreinigte Verdünnungsmittel kann nach Phasentrennung separiert und ebenfalls wenn nötig z. B. destillativ aufgearbeitet werden.

Die durch dieses Verfahren erhaltenen Polyisocyanat-Harze sind bei Raumtemperatur fest oder flüssig. Häufig werden sie in Lösemitteln gelöst.

Als Lösemittel können beliebige, in der Polyurethanchemie gebräuchliche Verdünnungsmittel wie beispielsweise Toluol, Xylol, Cyclohexan, Chlorbenzol, Butylacetat, Ethylacetat, Ethylglykolacetat, Pentylacetat, Hexylacetat, Methoxypropylacetat, Tetrahydrofuran, Dioxan, Aceton, N-Methylpyrrolidon, Methylethylketon, Testbenzin, höher substituierte Aromaten, wie sie beispielsweise unter der Bezeichnung Solvent Naphtha^{R}, Solvesso^{R}, Shellsol^{R}, Isopar^{R}, Nappar^{R} und Diasol^{R} im Handel eingesetzt werden. Gegebenenfalls können in den Lösemittelmischungen auch anteilig weniger polare Lösemittel wie Schwerbenzol, Tetralin, Dekalin und Alkane mit mehr als 6 Kohlenstoffatomen sowie Gemische derartiger Lösungsmittel enthalten sein. Die Konzentration der Polyisocyanate im Lösemittel wird hierbei auf 30 bis 95 Gewichts-%, vorzugsweise 50 bis 90 Gewichts-% Festkörper (FK) eingestellt, sofern überhaupt Lösemittel verwendet werden.

Weiterhin kann nach beendeter Oligomerisierungsreaktion wie z. B. Dimerisationsreaktion und/oder Trimerisationsreaktion noch eine weitere Modifizierung des Reaktionsprodukts mit z. B: niedermolekularen oder/und polymeren Hydroxylgruppen enthaltenden Verbindungen und/oder mit Blockierungsmitteln erfolgen.

Bei den durch das erfindungsgemäße Verfahren hergestellten Polyisocyanaten handelt es sich um wertvolle, unter dem Einfluß von Luftfeuchtigkeit aushärtbare Beschichtungsmaterialien. Bevorzugt werden sie als Vernetzer in 2-Komponentensystemen mit an sich bekannten isocyanatreaktiven Verbindungen eingesetzt. Hierzu zählen beispielsweise hydroxyfunktionelle Polyether, -ester, -amide, -carbonate, -acrylate, -butadiene bzw. Mischtypen oder Mischungen der genannten hydroxyfunktionellen Polymeren. Auch niedermolekulare Di- und Polyole, Di- und Trimerfettalkohole sowie aminofunktionelle Verbindungen können in 2K-Systemen Verwendung finden.

Mit blockierten isocyanatreaktiven Verbindungen können auch Einkomponentensysteme formuliert werden, ebenso können die nach dem erfindungsgemäßen Verfahren hergestellten Produkte auch in blockierter Form als oder in Beschichtungsmaterialien eingesetzt werden. Hierbei erfolgt die Trocknung bei höheren Temperaturen oberhalb beispielsweise 120 °C bis ca. 230 °C.

Neben den erfindungsgemäßen Verfahrensprodukten können in den Beschichtungen auch andere Hilfs- und Zusatzmittel wie beispielsweise die üblichen Benetzungsmittel, Verlaufsmittel, Hautverhinderungsmittel, Antischaummittel, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden.

Die erhaltenen Beschichtungsmaterialien können zur Beschichtung beliebiger Substrate wie beispielsweise Holz, Kunststoffe, Leder, Papier, Textilien, Glas, Keramik, Putz, Mauerwerk, Metalle oder Beton verwendet werden. Sie lassen sich mit üblichen Applikationsmethoden wie Spritzen, Streichen, Fluten, Gießen, Tauchen, Walzen aufbringen. Die Beschichtungsmittel können in Form von Klarlacken als auch in Form pigmentierter Lacke verwendet werden.

Die aus den erfindungsgemäßen Produkten hergestellten Beschichtungen härten bei 20 °C im allgemeinen während eines Zeitraums von einigen Minuten bis Stunden zu hochwertigen Überzügen aus. Die Härtung kann jedoch auch bei tieferen Temperaturen (bis -5 °C) oder beschleunigt bei höheren Temperaturen bis 200 °C erfolgen.

### Beispiele

Die nachfolgenden Beispiele und Vergleichsbeispiele sollen die Erfindung näher erläutern, ohne sie jedoch einzuschränken. Alle Angaben in "Teilen" und "%" beziehen sich auf das Gewicht. Der NCO-Gehalt wird gemäß DIN 53 185 bestimmt. Die dynamischen Viskositäten wurden bei 23 °C mit einem Rheometer MCR 51, Platte-Kegel Meßanordnung, der Fa. Anton Paar bestimmt. Durch Messung bei unterschiedlichen Schergeschwindigkeiten wurde sichergestellt, dass das Fließverhalten der beschriebenen erfindungsgemäßen Polyisocyanate dem idealer Newtonscher Flüssigkeiten entspricht. Die Angabe der Schergeschwindigkeit kann deshalb entfallen.

### Beispiel 1 (erfindungsgemäß)

In einem 1 Liter 4-Halsrundkolben mit aufgesetztem Kühler und Trockenrohr, Stickstoffeinleitung, Thermometer und Rühreinheit wurden 400 g Perfluoroktan zusammen mit 100 g HDI vorgelegt und auf 60 °C erwärmt. Hierbei wurde die Rühreinheit auf hohe Rührgeschwindigkeit (400 U/min) eingestellt, um eine feine Verteilung (Dispergierung) der Isocyanatkomponente in das Verdünnungsmittel zu bewirken. Nachdem sich eine konstante Temperatur von 60 °C eingestellt hatte, wurde die Reaktionsmischung durch Zusatz von 2 g Katalysatorlösung (Trimethylbenzylammoniumhydroxid 1,0 %ig in Methanol/2-Ethylhexanol) per Tropftrichter (über ca. 5 Minuten) zur Reaktion gebracht. Die Temperatur stieg innerhalb von 30 Minuten um 5 °C an. Es wurde bei dieser Temperatur weitergeführt. Die Reaktionsmischung blieb während der gesamten Reaktionszeit von 90 Minuten sehr dünnflüssig und somit leicht rührbar. Die Exothermie konnte gut durch Kühlung mit einem Wasserbad abgeführt werden. Bei Erreichen eines NCO-Gehalts der HDI-Phase von 39,0 % wurde die Reaktionsmischung durch Zugabe von 0,5 g Dibutylphosphat gestoppt. Im Anschluss wurde die Reaktionsmischung noch 2 Stunden nachgerührt und auf Raumtemperatur abgekühlt. Die Phasen wurden nach Abschalten des Rühres getrennt. Nach dünnschichtdestillativer Aufarbeitung der erhaltenen Polyisocyanatphase erhielt man ein Produkt mit folgenden Kenndaten:
NCO-Gehalt: 21,7 %
Viskosität: 2800 mPas
fr. HDI-Gehalt: 0,1 % (GC-Analyse)
Gehalt an Perfluoroktan: < 0,05 % (GC-Analyse)

### Beispiel 2 (nicht erfindungsgemäß, Vergleichsversuch)

Bei gleichen Bedingungen wie unter Beispiel 1 wurden 500 g HDI ohne Zusatz von Perfluoroktan bei 60 °C mit 10 g Katalysatorlösung zur Reaktion gebracht (gleiche Katalysatorkonzentration bez. auf HDI. Nach kurzer Inkubationszeit stieg die Temperatur innerhalb von 4 Minuten um fast 40 °C an und konnte nur mit Mühe mittels Wasserbad auf 60 °C gekühlt werden. Die Reaktionsmischung hatte sich gelblich verfärbt und war in der Viskosität deutlich angestiegen. Schon nach 30 Minuten war der NCO-Gehalt auf einen Wert von 31,6 % abgefallen. Die Reaktion wurde sofort durch Zugabe von 2 g Dibutylphosphat abgestoppt. Die Aufarbeitung der stark unterfahrenen Reaktionsmischung war nicht sinnvoll, da der niedrige NCO-Gehalt ein im Vergleich zu Beispiel 1) deutlich zu hochmolekulares und hochviskoses Produkt erwarten ließ.

Ein überhöhter Zusatz von Katalysator führt im erfindungsgemäßen Fall (Beispiel 1) zu keiner sicherheitskritischen Situation; die Temperatur kann weitestgehend konstant gehalten werden, die Viskosität und somit das Rührvermögen ändert sich nicht. Eine gute Wärmeaufnahme und -abfuhr ist jederzeit gegeben. Im Gegensatz hierzu erkennt man im nichterfindungsgemäßen Fall (Beispiel 2) trotz des kleinen Maßstabs (verbunden mit einer großen Oberfläche und damit guter Kühlung) einen großen negativen Temperatur- und Viskositätseffekt. Derartige Temperatursprünge sind im technischen Maßstab nicht zu beherrschen.

## Patentansprüche

1. Verfahren zur Herstellung von Polyisocyanaten, **dadurch gekennzeichnet, dass**
A) eine Isocyanatkomponente, welche zu mindestens 60 Gew.-% aus einem oder mehreren Diisocyanaten und zu maximal 40 Gew.-% aus einem oder mehreren Monoisocyanaten und/oder einem oder mehreren Isocyanaten mit einer Funktionalität ≥3 besteht
B) ggf. mit einem Katalysator B1) und/oder ggf. einer Reaktivkomponente B2)
in Form von Tropfen in einem Verdünnungsmittel C) bei einer Temperatur von 0 bis 200°C unter Oligomerisierung zur Reaktion gebracht wird, dann eine Phasentrennung erfolgt, vor, während oder nach der Phasentrennung die Reaktion ggf. durch Zusatz eines Katalysatorgifts D) unterbrochen wird, und anschließend das in der Isocyanatphase enthaltene Polyisocyanat vom überschüssigen monomeren Isocyanat bis auf einen Restgehalt von weniger als 1,0 Gewichts-% befreit wird, wobei die Restlöslichkeit der Isocyanatkomponente im Verdünnungsmittel unter 10 Gewichts-% liegt und als Verdünnungsmittel C) Alkane eingesetzt werden, die fluoriert oder perfluoriert sind.

2. Verfahren nach Anspruch 1, **dadurch gekennzeichnet, daß** als Komponente A) ausschließlich 1,6-Diisocyanatohexan, 1-Isocyanato-3,3,5-trimethyl-5-isocyanatomethylcyclohexan, 2,4'- und/oder 4,4'-Diisucyanatodicyclohexylmethan verwendet werden.

3. Verfahren nach Anspruch 1 oder 2, **dadurch gekennzeichnet, dass** in C) als Alkan Perfluoroktan eingesetzt wird.

4. Verfahren nach einem der Ansprüche 1 bis 3, **dadurch gekennzeichnet, dass** ein Gewichts-Verhältnis von Isocyanatkomponente A) zum Verdünnungsmittel C) von 1:10 bis 1:1 eingestellt wird.

5. Verfahren nach Aspruch 4, **dadurch gekennzeichnet, dass** das Gewichts-Verhältnis 1:5 bis 1:2 beträgt.

6. Verfahren nach einem der Ansprüche 1 bis 5 dadurch gekennzeiohnet, dass die Reaktion kontinuierlich durchgeführt wird.

## Claims

1. Process for producing polyisocyanates, **characterized in that**
A) an isocyanate component composed of at least 60% by weight of one or more diisocyanates and not more than 40% by weight of one or more monoisocyanates and/or one or more isocyanates having a functionality ≥ 3
B) optionally with a catalyst B1) and/or optionally a reactive component B2)
is reacted in the form of droplets in a diluent C) at a temperature of 0 to 200°C with oligomerization, then a phase separation takes place; before, during or after the phase separation, the reaction is interrupted optionally by addition of a catalyst poison D); and subsequently the polyisocyanate present in the isocyanate phase is freed from the excess monomeric isocyanate down to a residual content of less than 1.0% by weight, the residual solubility of the isocyanate component in the diluent being below 10% by weight and alkanes which are fluorinated or perfluorinated being used as diluents C).

2. Process according to Claim 1, **characterized in that** exclusively 1,6-diisocyanatohexane, 1-isocyanato-3,3,5-trimethyl-5-isocyanatomethylcyclohexane, 2,4'- and/or 4,4'-diisocyanatodicyclohexylmethane are used as component A).

3. Process according to Claim 1 or 2, **characterized in that** perfluorooctane is used as alkane in C).

4. Process according to any one of Claims 1 to 3, **characterized in that** a weight ratio of isocyanate component A) to diluent C) of 1:10 to 1:1 is set.

5. Process according to Claim 4, **characterized in that** the weight ratio is 1:5 to 1:2.

6. Process according to any one of Claims 1 to 5, **characterized in that** the reaction is carried out continuously.

## Revendications

1. Procédé pour la préparation de polyisocyanates, **caractérisé en ce qu'**on fait réagir, avec oligomérisation,
A) un composant isocyanate, qui est constitué à raison d'au moins 60% en poids d'un ou de plusieurs diisocyanates et à raison d'au maximum 40% en poids d'un ou de plusieurs monoisocyanates et/ou d'un ou de plusieurs isocyanates présentant une fonctionnalité ≥ 3
B) le cas échéant avec un catalyseur B1) et/ou le cas échéant un composant réactif B2)
sous forme de gouttes dans un diluant C) à une température de 0 à 200°C, puis une séparation de phases a lieu, la réaction est arrêtée avant, pendant ou après la séparation des phases, le cas échéant par addition d'un poison de catalyseur D), puis le polyisocyanate contenu dans la phase isocyanate est libéré de l'isocyanate monomère en excès jusqu'à une teneur résiduelle inférieure à 1,0% en poids, la solubilité résiduelle du composant isocyanate dans le diluant étant inférieure à 10% en poids et en utilisant, comme diluant C), des alcanes, qui sont fluorés ou perfluorés.

2. Procédé selon la revendication 1, **caractérisé en ce qu'**on utilise comme composant A) exclusivement du 1,6-diisocyanatohexane, du 1-isocyanato-3,3,5-triméthyl-5-isocyanatométhylcyclohexane, du 2,4'-diisocyanatodicyclohexylméthane et/ou du 4,4'-diisocyanatodicyclohexylméthane.

3. Procédé selon la revendication 1 ou 2, **caractérisé en ce qu'**on utilise dans C), comme alcane, du perfluorooctane.

4. Procédé selon l'une quelconque des revendications 1 à 3, **caractérisé en ce qu'**on règle un rapport pondéral du composant isocyanate A) au diluant C) de 1:10 à 1:1.

5. Procédé selon la revendication 4, **caractérisé en ce que** le rapport pondéral vaut 1:5 à 1:2.

6. Procédé selon l'une quelconque des revendications 1 à 5, **caractérisé en ce que** la réaction est réalisée en continu.
